# EUROPEAN PATENT SPECIFICATION

(11) **EP 4 532 036 B1**
(45) Date of publication and mention of the grant of the patent: **04.03.2026**
(21) Application number: 23728348.6
(22) Date of filing: 24.05.2023
(51) Int. Cl.: A61Q 11/02, A61K 8/04, A61K 8/02, A61K 8/25, A61K 8/49, A61K 8/73, A61K 8/81

(54) **SPHERICAL GRANULES OF CLAY-BASED ANTIMICROBIAL PARTICLES**
KUGELFÖRMIGE GRANULATE AUS ANTIMIKROBIELLEN TONBASIERTEN PARTIKELN
GRANULES SPHÉRIQUES DE PARTICULES ANTIMICROBIENNES À BASE D'ARGILE

(30) Priority: 31.05.2022 EP 22176301
(43) Date of publication of application: 09.04.2025
(73) Proprietor: Unilever IP Holdings B.V., 6708 WH Wageningen (NL); Unilever Global IP Limited, Wirral, Merseyside CH62 4ZD (GB)
(72) Inventor: AMIN, Purnima, Dhanraj, 6708 WH Wageningen (NL); CHANDRASEKARAN, Sembian, 6708 WH Wageningen (NL); JAIN, Divya, Dineshkumar, 6708 WH Wageningen (NL); KORANNE, Ketki, Yogesh, 6708 WH Wageningen (NL); RAWOOL, Ajit, Ganesh, 6708 WH Wageningen (NL); SRIVASTAVA, Madalasa, 6708 WH Wageningen (NL)
(74) Representative: Tansley, Sally Elizabeth
(86) International application number: PCT/EP2023/063865
(87) International publication number: WO 2023/232571

(56) References cited:
- WO-A1-2021/259748
- SHIMABAYASHI ET AL: "Interaction between hydroxypropylcellulose and surfactant and its effect on dispersion stability of kaolinite suspension in an aqueous phase", PROGRESS IN COLLOID & POLYMER SCIENCE (1997), 106, 136-140 CODEN: PCPSD7; ISSN: 0340-255X, 1 January 1997 (1997-01-01), XP055980342

## Description

### Field of the Invention

This invention is defined in the appended claims.

### Background of the Invention

A variety of cosmetic and homecare compositions are known for many end-use benefits through agents that include fragrances, antimicrobial agents, and sunscreens. At times the benefit agents are encapsulated or granulated to make them, for e.g., more effective, or more stable.

WO2011036031 A1 (Unilever) discloses a bipolar antimicrobial particle, in which precursor is an asymmetric 1:1 or 2:1:1 clay particle comprising alternating tetrahedral and octahedral sheets terminating with a tetrahedral sheet at one external surface plane and an octahedral sheet at another external surface plane, with an antimicrobial group attached to the coordinating cation on one of the said external surface plane selected from a quaternary ammonium compound comprising a single alkyl or alkenyl long chain having an average chain length greater than or equal to C20, or a quaternary ammonium material selected from Cetylpyridinium chloride (CPC), Cetyltrimethylammonium Chloride (CTAC), Cetyltrimethylammonium Bromide (CTAB) Benzalkonium Chloride (BKC), Benzethonium chloride, cetrimide, Quaternium, polyhexamethylene BH, antimicrobial alcohols, antimicrobial phenols, antimicrobial organic acids/salts, Zinc pyrithione, Ketoconazole, Octopirox^{®} or combinations thereof. The quaternary ammonium compound having antimicrobial properties is immobilized on clay which acts as a carrier.

WO2014/102032 A1 (Unilever) discloses a toothpaste comprising the aforesaid bipolar antimicrobial particle.

US2012121669 A1 (Colgate-Palmolive) discloses oral care compositions comprising capsules. US2008242767 A1 (Colgate) discloses polymeric microcapsules for a dentifrice. The microcapsules stay intact during manufacture and storage of the toothpaste. The microcapsules of 5 to 50 µm are said to possess good mechanical properties to sustain shear forces up to about 1000/s during mixing and high-speed filling; good temperature stability that allow the capsules to stay intact at temperatures up to about 70°C; good chemical stability allowing the capsules to be stable in the presence of sodium lauryl sulfate and pH 5 to 8. The microcapsules break and release CPC to the oral cavity directly without formation of inactive complex.

KR20040081936 A (Dong A Pharm, 2004) discloses a toothpaste containing CPC granules, i.e., 0.01 to 5 wt% CPC based on total weight of the composition, in which the CPC is coated on the surface of granules made of precipitated calcium carbonate, silica, zeolite, colloidal silica dioxide and anhydrous calcium phosphate. Particle size of the granules is 100 to 750 µm and a strength of 150 to 650 g/cm⁻².

WO2021/259748 A1 (Unilever) discloses an oral care composition having 0.1 to 10 wt.% bipolar antimicrobial particle and a compound of zinc.

It has been determined that the bipolar antimicrobial material disclosed in WO2011036031 A1 and WO2014/102032 A1 is suited for use in a variety of home and personal care products, particularly oral care products per se, but we have observed that when formulated into a viscous flowable composition like a toothpaste, which is usually packaged in collapsible tubes, the bipolar antimicrobial material is not distributed as uniformly across the length and breadth of the packaged composition as would be reasonably expected. There is spatial segregation forming zones or regions that are rich in the material, and thereby the antimicrobial material per-se, and correspondingly there are regions of lesser concentration. In addition, at least for the transparent or water-white formulation, the powdery or amorphous form of the bipolar antimicrobial material may pose problems with visual appearance of the compositions. Such visual appearance may be corrected by use of masking agents but the problem of inequal distribution or spatial segregation cannot be solved by any masking agent.

A standard solution is the use of rheological additives to arrest the free movement of the bipolar antimicrobial material, but it is not advisable due to the unavoidable effects on the viscosity of such compositions.

As a result, there is a risk of uneven distribution of the antimicrobial material across the bulk of the home or personal care composition. Therefore, certain users may dispense and get an efficacious portion of the composition while certain other users might not get an efficacious portion and may never know the reasons whilst be the under the impression that the portion is efficacious as is a true representation of the bulk of the composition.

An object of the invention is to overcome at least one drawback, limitation, or disadvantage of prior art.

### Summary of the Invention

Disclosed is a granular composition comprising 30 wt.% to 70 wt% bipolar antimicrobial particle, where said antimicrobial particle comprises a clay and a quaternary ammonium compound, in which said clay is an asymmetric 1:1 or a 2:1:1 clay having alternating tetrahedral and octahedral sheets, terminating with a tetrahedral sheet at one external surface plane and an octahedral sheet at another external surface plane, each said sheet comprising a co-ordinating cation; and said quaternary ammonium compound is attached to said coordinating cation on at least one external surface plane, wherein granules of said composition are spherical, having sphericity of 0.7 to 1.0.

In accordance with a second aspect disclosed is a liquid personal care composition comprising granules of the granular composition of first aspect.

When the granular composition of the invention is included in a liquid composition, especially a viscous liquid homecare or a viscous liquid personal care composition, the granules remain uniformly suspended in the matrix of the composition thereby reducing the possibility of spatial segregation of such granules which is often observed in case of such compositions.

The present inventors have found that when the bipolar antimicrobial particle is incorporated in a granular composition where the granules of the composition are spherical, having a sphericity ranging from 0.7 to 1.0, the granule of the granular composition were found to retain their shape and size when incorporated in the personal care composition. It was surprisingly found that when the granular composition with granules having specific sphericity according to the present invention is formulated into a personal care composition it provides even distribution of the antimicrobial particle across the bulk of the personal care composition. This ensures that users get an efficacious portion of the personal care composition which is representative of the bulk composition in each dispense.

### Detailed Description of the Invention

"Length", "width" and "thickness" refers to the length, width and thickness of particles or microcapsule in an unaggregated state. The term "length" refers to the average dimension of a granule typically along the longitudinal axis. The term "width" refers to the average dimension of the granule perpendicular to the length and typically perpendicular to the longitudinal axis. The terms "thickness" refers to the average dimension of the granule that is perpendicular to the length and width. The length, width and thickness may be measured for example by scanning electron microscopy (SEM) by averaging the values of at least ten particles.

"Sphericity" is the measure of how closely the shape of an object approaches that of a mathematically perfect sphere. Sphericity of granules is the ratio of the surface area of a sphere (with the same volume as the given particle) to the average surface area of the particles. Sphericity may be determined as follows.

An SEM image of the silica particle sample is magnified 20,000 times, which is representative of the silica particle sample, and is imported into photo imaging software, and the outline of each particle (two-dimensionally) is traced. Particles that are close in proximity to one another but not attached to one another should be considered separate particles for this analysis. The outlined particles are then filled in with color, and the image is imported into particle characterization software (e.g., IMAGE-PRO PLUS available from Media Cybernetics, Inc., Bethesda, Md.) capable of determining the perimeter and area of the particles.

"Specific surface area" as used herein refers to specific surface area determined according to Brunauer-Emmett-Teller method. The value of the specific surface area was measured by meeting the requirements set out in ASTM standard D 3663-78.

"Particle size" as used herein refers to particle diameter in non-aggregated state unless otherwise stated. For polydisperse samples having particulate with diameter no greater than 1 µm, diameter means the z-average particle size measured, for example, using dynamic light scattering (see international standard ISO 13321) with an instrument such as a Zetasizer Nano^{™} (Malvern Instruments Ltd, UK). For polydisperse samples having particulate with diameter greater than 1 µm, diameter means the apparent volume median diameter (D50, also known as x50 or sometimes d(0.5)) of the particles measurable for example, by laser diffraction using a system (such as a Mastersizer^{™} 2000 available from Malvern Instruments Ltd) meeting the requirements set out in ISO 13320.

The term granular means that the composition of the invention is in the form of granules. The term granular also means that the composition is neither amorphous nor powdery.

### The invention

The present invention relates to a granular composition comprising 30 wt.% to 70 wt.% bipolar antimicrobial particle, where said antimicrobial particle comprises a clay and a quaternary ammonium compound, in which said clay is an asymmetric 1:1 or a 2:1:1 clay having alternating tetrahedral and octahedral sheets, terminating with a tetrahedral sheet at one external surface plane and an octahedral sheet at another external surface plane, each said sheet comprising a co-ordinating cation; and said quaternary ammonium compound is attached to said coordinating cation on at least one external surface plane, wherein granules of said composition are spherical, having sphericity of 0.7 to 1.0.

The term "granular composition" means that the composition is in the form of granules.

It is preferred that granules of the granular composition of the invention are nearly spherical. However, some degree of non-sphericity is not detrimental to its efficacy. Sphericity of granules is from 0.7 to 1.0, more preferably 0.75 to 1.0, still more preferably 0.8 to 1.0. Sphericity of 1.0 means perfectly spherical granules. Without wishing to be bound by theory it is believed that when sphericity is 0.7 to 1.0, the granules are more efficacious. This is especially true when the granules are used in the viscous personal care composition.

It is preferred that bulk density of the granular composition is from 0.800 to 0.950 g/cm³. Further preferably the average particle size of the granules is 650 µm to 900 µm.

It is preferred that the granules of the granular composition are friable and friability is from 0.6 to 0.9 w/w.

In the particularly preferred that in the composition of the invention the granules are insoluble in the medium of the composition into which the granular composition is incorporated. In this context, "insoluble" means having sufficient insolubility at ambient temperature that the granules remain undissolved or substantially undissolved in the composition such that their friability under the conditions of use of the composition and thus their ability to perform their intended function are not deleteriously affected.

### The bipolar antimicrobial particle and the clay

The precursor of the particle with bipolar topospecific characteristics according to the present invention is an asymmetric 1:1 or 2:1:1 clay particle having alternating tetrahedral and an octahedral sheets terminating with a tetrahedral and an octahedral sheet at exterior surface planes. Particle of 1:1 clay is particularly preferred as precursor.

It is preferred that the 1:1 clay is from kaolinite or serpentine subgroup of minerals. The species included within the kaolinite subgroup include but are not limited to kaolinite, dickite, halloysite and nacrite. The species within the serpentine subgroup include but are not limited to chrysolite, lizardite, and amesite.

It is preferred that 2:1:1 clay includes chlorite group of minerals. The chlorite comprises tetrahedral-octahedral-tetrahedral sheets like 2:1 clays, with an extra weakly bound brucite like layer between tetrahedral layers.

The tetrahedral sheet preferably comprises coordinating tetrahedral cations of silicon. The tetrahedral sheet may also comprise isomorphously substituted coordinating tetrahedral cations which are not silicon. Isomorphously substituted coordinating tetrahedral cations include, but are not limited to, cations of aluminum, Iron or boron.

The octahedral sheet preferably comprises coordinating octahedral cation of aluminum. The octahedral sheet may also comprise isomorphously substituted coordinating octahedral cations which are not aluminium. Isomorphously substituted coordinating octahedral cations include cations of magnesium or iron.

It is preferred that the antibacterial quaternary ammonium compound is attached to the coordinating cations on the exterior side of one of the external surface planes. Accordingly, the antimicrobial molecule is attached to coordinating cations on the exterior side of the tetrahedral sheet. Alternatively, the antimicrobial molecule is attached to the coordinating cations on the exterior side of the octahedral sheet. According to a further aspect, coordinating cations on the exterior side of each of the tetrahedral and the octahedral surface sheets are attached to a antimicrobial molecule, with the proviso that the antimicrobial molecule attached to the coordinating cations on the exterior side of the tetrahedral surface sheet is not identical to the molecule attached to the coordinating cations on the exterior side of the octahedral surface sheet. The antimicrobial molecule is preferably attached to the coordinating cations on the external surface of the octahedral surface plane and is not preferably attached to coordination cations of non-exterior tetrahedral or octahedral plane or on the interior side of the surface sheets.

It is preferred that in the antimicrobial material, the antibacterial agent is attached to coordinating cations on the external surface of the octahedral surface plane.

In the granular composition of the invention, it is preferred that, on w/w basis, said bipolar antimicrobial particle comprises 0.5 parts to 5 parts by weight of said quaternary ammonium compound and 90 parts to 98 parts by weight of said clay.

It is preferred that the quaternary ammonium compound is cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide.

It is particularly preferred that the antimicrobial agent is cetylpyridinium chloride (CPC). Without being bound by theory it is believed that the primary activity is linked to the cationic charge of its amine group. Thus, cetylpyridinium chloride is attracted to and binds to the negatively-charged protein moieties on the cell membrane or cell wall of microorganism and to a surface, example, teeth, which are also typically negatively charged. The resulting attachment to microorganisms disrupts the cell wall structure causing leakage of the intracellular fluids, eventually killing the associated microorganism.

It is particularly preferred that precursor of the clay is a 1:1 clay particle. Further preferably the precursor of the clay is kaolinite. It is particularly preferred that the quaternary ammonium compound is cetyl pyridinium chloride. It is further particularly preferred that when the precursor of the clay is kaolinite, the antimicrobial quaternary ammonium compound is cetyl pyridinium chloride.

### Process for preparing a bipolar antimicrobial particle

Any chemical reaction or series of reactions wherein an antibacterial quaternary ammonium compound is attached selectively to coordinating cations on the exterior plane of either the tetrahedral or the octahedral surface plane of asymmetric clay can be used to prepare the bipolar antimicrobial particle. In order to obtain a true bipolar antimicrobial particle it is preferred that the reaction is selective to only one of the exterior planes. By selective is meant that more than 50% of the total antimicrobial molecule is present on one of the exterior planes, preferably more than 75%, more preferably than 80%, still more preferably than 90%, even more preferably than 95%, or even more than 99%. The chemical reaction or series of reactions wherein the same antimicrobial molecule attached to coordinating cations of both the surface sheets, viz octahedral and tetrahedral, are therefore not preferred.

The particle with bipolar characteristics may have two distinct regions on its surface having non-identical surface characteristics. It is particularly preferred that the particle has two spatially distinct exterior faces having distinct surface characteristics. It is envisaged that by selecting specific antimicrobial molecule having specific group, and selectively attaching them to coordinating cations of tetrahedral and/or octahedral surface sheets, it is possible to impart anisotropic characteristics of various types to the surface of particle with bipolar characteristics.

The process comprises the steps of contacting the precursor with a mineral acid, adding a antibacterial quaternary ammonium compound to the mixture, adjusting the pH of the solution above 8, heating the mixture to temperature of 50 to 150°C for about 30 minutes to 10 hours while stirring, and separating the solid product comprising bipolar particulate antimicrobial. When temperature is greater than 100°C is applied, a pressure vessel is preferred.

It is preferred that at first the raw clay is treated with a mineral acid preferably hydrochloric acid. The hydrochloric acid is used in a concentration range of 0.01 N to 1 N, preferably about 0.1 N. The clay particle with the acid is then stirred. The stirring is typically done for 10 to 60 minutes, preferably about 30 minutes.

After treating the precursor with mineral acid for about 30 minutes, pH of the system is preferably adjusted to above 8 by adding 0.1 (M) NaOH to the solution.

After treating the clay with hydrochloric acid it is preferred that the desired antimicrobial molecules were added to the dispersion. The antimicrobial molecules are then added in a concentration of 0.001 to 30 % of the total weight of the dispersion, preferably 0.01 to 5%.

After adding the antimicrobial molecule and adjusting the pH of the dispersion, the solution is preferably heated for between 1 to 10 hours, preferably 4 to 8 hours and more preferably about 6 hours while stirring at 50°C to 150°C preferably 70°C to 90°C more preferably at 80°C while stirring. Separating the solid product comprising the bipolar particulate antimicrobial.

After the reaction, the dispersion mixture is preferably centrifuged to obtain the bipolar antimicrobial particle as residue. Then it is preferably washed with copious amount of water and subsequently with a ketone solvent (e.g., acetone). After that it is dried in an oven to get the final product.

In this reaction, antibacterial quaternary ammonium compound is attached to the coordinating cations of the octahedral sheet preferable by covalent bonding. The antimicrobial particle made by this process has different wettability characteristics for two external surface planes.

It is preferred that the quaternary ammonium compound is selected from cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide.

It is particularly preferred that the antibacterial quaternary ammonium compound is cetylpyridinium chloride (CPC). It is believed that the primary activity is linked to the cationic charge of its amine group. Thus, cetylpyridinium chloride is attracted to and binds to negatively-charged protein moieties on the cell membrane or cell wall of a microorganism and to a surface, example, tooth surfaces, which are also typically negatively charged. The resulting attachment to microorganisms disrupts the cell wall structure causing leakage of the intracellular fluids, eventually killing the associated microorganism.

It is particularly preferred that precursor of the clay is a 1:1 clay particle. Further preferably the precursor of the clay is kaolinite. It is particularly preferred that the antimicrobial quaternary ammonium compound is cetyl pyridinium chloride. It is further particularly preferred that when the precursor of the clay is kaolinite, the antimicrobial quaternary ammonium compound is cetyl pyridinium chloride.

It is preferred that median particle size (D50) of the bipolar antimicrobial particle is 0.1 to 10 µm, more preferably 0.4 to 1 µm and most preferably 0.5 to 0.8 µm. Lower particle size also provides an effective mechanism for increased the loading of the antibacterial agent, if so desired. Particle size distribution (D50) is also known as the median diameter or the medium value of the particle size distribution, it is the value of the particle diameter at 50 percent in the cumulative distribution. For example, if D50 is 5.8 µm, then 50 % of the particles in the sample are larger than 5.8 µm and 50 percent smaller than 5.8 µm. D50 is usually used to represent the particle size of group of particles. The D50 is the size in microns that splits the distribution with half above and half below this diameter.

Lower particle size also provides an effective mechanism for increased the loading of the antibacterial quaternary ammonium compound, if so desired. Particle size distribution (D50) is also known as the median diameter or the medium value of the particle size distribution, it is the value of the particle diameter at 50 percent in the cumulative distribution. For example, if D50 is 5.8 µm, then 50 % of the particles in the sample are larger than 5.8 µm and 50 percent smaller than 5.8 µm. D50 is usually used to represent the particle size of group of particles. The D50 is the size in microns that splits the distribution with half above and half below this diameter.

### Size and sphericity

The granules of the composition are spherical having sphericity of 0.7 to 1.0. However, some degree of non-sphericity is not detrimental to the performance of the granular composition. More preferably the sphericity is 0.75 to 1.0, still more preferably 0.8 to 1.0. Sphericity of 1.0 means perfectly spherical granules. Without wishing to be bound by theory it is believed that when sphericity is 0.7 to 1.0, the granules are more efficacious then when outside this range. This is especially true when the granules are used in the viscous liquid homecare and personal care compositions.

Preferably the bulk density of the granular composition is from 0.800 to 0.950 g/cm³. Further preferably the average particle size of the granules is 650 to 900 µm.

It is preferred that the granules of the granular composition are friable having friability from 0.6 to 0.9 w/w. In the particularly preferred compositions of the invention the granules should be insoluble in the medium of the composition into which it is incorporated. In this context, "insoluble" means having sufficient insolubility at ambient temperature that the granules remain undissolved or substantially undissolved in the composition such that their friability under the conditions of use of the composition and thus their ability to perform their intended function are not deleteriously affected.

### Binder

The granular composition of the invention preferably comprises 10 wt.% to 40 wt% of a water-insoluble binder.

As used herein, "binder" means a material used to bind together two or more other materials in the granule. Preferably the binder is a carbohydrate. It is preferred that the carbohydrate is starch, more preferably corn starch. When the water-insoluble binder is starch it is preferred that the amount that the amount of the starch in the composition ranges from 10 wt.% to 20 wt.%, still more preferably from 10 wt.% to 19 wt.% and further more preferably from 10 wt.% to 18 wt.%. The binder according to the present invention is a carbohydrate which is not a cellulose or a cellulose derivative.

Preferably, the granular composition comprises from 10 wt.% to 40 wt.% water-insoluble binder, more preferably from 10 wt.% to 30 wt.% water-insoluble binder. Preferably the granular composition comprises at least 11 wt.%, still preferably at least 12 wt.%, still preferably at least 13 wt.%, most preferably at least 15 wt.% of water-insoluble binder, but typically not more than 35 wt.%, still preferably not more than 30 wt.%, still further preferably not more than 28 wt.%, still more preferably not more than 27 wt.% and most preferably not more than 25 wt.%, still more preferably not more than 20 wt.% of binder based on the weight of the granular composition.

Preferably when the granular composition includes starch as the binder, it is preferred that the composition includes from 10 wt.% to 20 wt.% starch.

### Coating agent:

The granular composition of the invention preferably comprises 5 wt.% to 23 wt.% water-insoluble coating agent. Preferably the coating agent is selected from the group consisting of [meth]acrylate, aminoalkyl [meth]acrylate, cellulose or a derivative of cellulose or mixtures thereof. The coating agent may also serve as a spheronizer. An example of the spheronizer in the composition of the present invention is preferably microcrystalline cellulose. It is preferred that the coating agent is based on [meth]acrylates. It is preferred that the coating agent based on [meth]acrylates is a latex dispersion of a neutral polymer produced by emulsion polymerization of, example, ethyl acrylate and methyl methacrylate. Commercially available coatings agents include Eudragit NE 30 D and Eudragit NM 30 D whic are polymethacrylate-based coating systems. Difference between Eudragit NE 30 D and Eudragit NM 30 D is in the content and nature of emulsifier.

It is also preferred that the coating agent is a cellulose or a cellulose derivative. Preferably the coating agent is a fibrous cellulose. More preferably the binder is an alkyl cellulose, still more preferably the alkyl cellulose is ethyl cellulose. Ethyl cellulose polymer, as used herein, means a derivative of cellulose in which some of the hydroxyl groups on the repeating glucose units are converted into ethyl ether groups. Preferably the coating agent may be in any form including but not limited to solid, liquid dispersion more preferably an aqueous dispersion or mixtures thereof.

Preferably when the coating agent is cellulose or a cellulose derivative it is preferred that the amount of cellulose or a cellulose derivative is in an amount ranging from 5 wt.% to 11 wt.%, still more preferably from 5 wt.% to 10.5 wt.%. Not wishing to be bound by theory, the inventors believe that when the amount of the cellulose or cellulose derivative in the granular composition is in an amount higher than 11 wt.% it causes the granular composition to swell in presence of water, particularly when the granular composition according to the present invention is incorporated into an oral care composition. This swelling over time negatively impacts the size and shape of the granular composition during the storage.

Preferably the coating agent has a low glass transition temperature, more preferably the glass transition temperature is less than 132 °C, more preferably from 40 °C to 50 °C.

When the binder and the coating agent are both based on[meth]acrylate polymer, it is preferred that they are not identical.

Preferably, the granular composition comprises from 5 wt.% to 23 wt.% coating agent, more preferably from 5 wt.% to 15 wt.% coating agent. Preferably the granular composition comprises at least 6 wt.%, still preferably at least 8 wt.%, still preferably at least 9 wt.%, most preferably at least 10 wt.% of coating agent, but typically not more than 19 wt.%, still preferably not more than 18 wt.%, still further preferably not more than 17 wt.%, still more preferably not more than 16 wt.% and most preferably not more than 15 wt.%, of coating agent based on the weight of the granular composition.

It is further preferred that the granular composition of the invention comprises at least one of a granulating aid or a spheronizing aid.

### Process for preparing the granular composition:

According to another aspect of the present invention disclosed is a process for preparing the granular composition according to the first aspect.

A process for preparing the granular composition according to the first aspect of the present invention, the process comprising the steps of:
(i) admixing a bipolar antimicrobial particle, water-insoluble binder, and a water-insoluble coating agent to form a mixture;
(ii) extruding the mixture to form an extrudate;
(iii) spheronizing the extrudate to form the granular composition,
wherein the granule of said composition are spherical, having sphericity of 0.7 to 1.0.

According to the second aspect the process for preparing the granular composition includes the step of admixing the bipolar antimicrobial particle, water-insoluble binder, and water-insoluble coating agent to form a homogeneous mixture. Preferably the admixing is carried out in a mixer. Preferably the mixer may be any mixer known to the person skilled in the art for granulating. Preferably the ingredients are mixed in the mixer to form a wet mass of desired consistency.

The wet mass is then preferably extruded through a die slot of an extruder and the extrudate emerging from the die slot is cut. Preferably the wet mass is extruded through, for example, a screw type extruder. Although the die slot and therefore the extrudate may take any suitable shape, spaghetti or noodle shape is more preferred. When the extrudate takes the form of spaghetti or noodle, the extruder die hole diameter is preferably in the range of about 0.1 mm to about 1 mm, more preferably from 0.4 to 0.8 mm. The extrudate is preferably cut after passing through the die-plate of the extruder using a high-speed cutter set.

Once the extrudate has been converted into plural smaller sized pellets, the next step involves spheronizing the extrudate in a spheronizer to render the pellets spherical. The spheronizer generally deforms the pellets by a combination of mechanical forces and optional heat. When heat is applied to the pellets being spheronized, preferably the temperature is at least 1 to 5 degrees above the Tg or the melting point of the most abundant thermoforming material in the pellets.

Preferably after spheronization, the spheronized extrudate is dried and then preferably sieved to provide the granule composition according to the present invention. Preferably the drying step is carried out at a temperature of 40° to 60°C. Extrusion/spheronization is a process where the active ingredient and excipients are sequentially blended, wet-granulated, extruded and spheronized to form pellets.

### Personal Care Compositions

The invention also relates to a liquid personal care composition comprising the granular composition of the invention. Preferably the personal care composition is a leave-on skin lotion, cream, antiperspirant, deodorant, serum, shampoo, hair conditioner, hair serum, hair gel, dentifrice, handwash, bodywash, shower gel or soap.

It is preferred that the liquid personal care composition is a dentifrice. Preferably the dentifrice is a toothpaste. Further preferably the toothpaste is a gel. Usually such toothpastes are transparent. It is preferred that when the dentifrice is a toothpaste, the toothpaste comprises at least one of calcium-based abrasive or silica-based abrasive, more preferably a silica based abrasive. Preferably, the abrasive silica is precipitated silica. Suitable precipitated silicas for use as abrasive amorphous silica particles in the invention are commercially available and include those marketed by PQ Corporation under the trade names SORBOSIL^{®} AC 43, AC77, AC35 and SORBOSIL^{®} AC 33. Mixtures of any of the materials may also be used. Preferably the toothpaste composition comprises 1 to 10 wt% abrasive silica.

Alternatively, the toothpaste comprises a calcium-based abrasive. A particularly preferred abrasive is fine ground natural chalk (FGNC). It is obtained from limestone or marble. FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion. Other preferred calcium containing abrasives include dicalcium phosphate (DCP), calcium pyrophosphate and precipitated calcium carbonate (PCC). When a combination of Calcium containing abrasives is used, it is preferred that FGNC is 35 to 100 %, more preferably 75 to 100 % and especially from 95 to 100 % of the total abrasive. In such cases, the balance, most preferably, is PCC.

Preferably the toothpaste comprises a liquid continuous phase from 40 to 85% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice).

Humectants are generally included in toothpastes for a soft, supple mouth feel. Humectants also reduce the tendency of toothpastes to lose moisture. Preferred toothpaste compositions contain 3.5 wt.% to 40 wt.% humectants. Further preferred compositions have 10 wt.% to 40 wt.%, more particularly 10 wt.% to 20 wt.% humectants. A particularly preferred humectant is sorbitol, generally available as 70% aqueous solution. Other preferred humectants include glycerine, maltitol and xylitol. More preferred toothpastes contain glycerine and sorbitol for a lubricated mouth feel, but their cumulative levels should not exceed the disclosed upper limit. Lower humectant content provides an effective way to reduce the cost of the product.

It is preferred the toothpaste comprises a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

It is further preferred that the toothpaste of the invention comprises a surfactant in an amount of from 0.2 to 10% by weight based on the total weight of the dentifrice. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Further preferably the toothpaste comprises a smectite clay which is in addition to the clay present by way of the bipolar antimicrobial particle. Smectites constitute a group in the class of natural aluminosilicate minerals known as phyllosilicates or layered silicates. Preferred smectite clay is selected from montmorillonites (bentonites, hectorites and derivatives thereof); purified aluminium magnesium silicates (various grades are commercially available as VEEGUM^{(R)}from R. T. Vanderbilt Company); purified sodium magnesium silicates (commercially available as LAPONITE^{(R)} in various grades); organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite/ and mixtures thereof. Aluminium magnesium silicate clays are particularly preferred. An example is VEEGUM^{(R)} HV. The clay tends to swell when exposed to water. Preferred toothpaste compositions contain 0.2 to 3 wt percent clay. More preferred compositions include 0.5 to 1 wt percent clay.

The smectite clay not only plays a role in sensory profile as is believed, but it also plays a role in thickening the composition.

Further preferably the toothpaste comprises a zinc salt, preferably zinc sulphate or zinc chloride, more preferably zinc sulphate heptahydrate. Preferably the level of zinc salt is from 0.05 to 1.0 wt% of the total composition more preferably from 0.1 to 0.5 wt%.

Compositions of the invention may comprise a preservative, a preferred preservative is sodium benzoate. Preferably the level of preservative is from 0.1 to 1 wt% of the total composition. It is preferred that pH of the composition at 20 °C is from 4 to 10, more preferably 7 to 9.

The compositions of the invention may also comprise a deposition aid. The term "deposition aid" in the context of this invention generally means a material which further aids deposition of whitening agents from the composition. An example is polystyrene sulphonates. Another example is Gantrez^{®} polymers.

The dentifrice composition may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

Preferably the personal care composition includes from 0.1 wt.% to 3 wt.% of the granular composition according to the present invention, still more preferably the personal care composition includes from 1 wt.% to 2.5 wt.% of the granular composition.

Various examples illustrative of the invention are presented as follows and are no way to be considered as limiting the scope of the invention. The invention will now be explained with help of the following non-limiting examples.

### Examples

### Example 1: Preparation of antimicrobial particle (CPC-clay)

Five grams Kaolinite was taken in a 500 ml of 0.1N HCl solution and sonicated for 30 minutes. The pH was then increased to 9 by addition of NaOH solution drop wise. To this 10 g CPC (cetyl pyridinium chloride) was added and the suspension was stirred over a magnetic stirrer for six hours while maintaining the temperature of the solution at 75 to 80°C. The suspension was then washed with water for about ten times to remove the excess CPC and a final ethanol wash was given. The clay was then dried in a hot air oven. The composition prepared as above contained 97.5 wt% kaolinite and 2.5 wt% cetyl pyridinuim chloride. D50 of the particles was 0.5 µm.

### Example 2

In this experiment the bipolar antimicrobial particle of Example 1 was granulated following a process as briefly described below.

All the raw materials were weighed as per the requirement of the formulation concerned.

The excipients, if any, were blended and mixed well in a Planetary Mixer for 2 to 5 minutes at low rpm. Then the granulating fluid was added in small amounts to the dry blend and allowed to mix thoroughly. The process steps were repeated until a wet mass of required consistency was obtained. Then the wet mass was extruded at a speed of 50 rpm using 0.8 mm die. The extrudate was spheronized, ensuring a balance between spheronization speed, time and spheronizer plate dimensions. The resultant pellets were dried at 60°C until all the pellets were completely dry. The pellets were sieved to obtain granules within defined size limits.

### Example 3: Comparative examples

Granules of varying composition were prepared for comparative analysis. Details of the compositions are shown in Table 1. All the granular compositions of Table 1 were comparative examples, i.e., not within scope of the present invention.

**TABLE 1**

| **Ingredients/wt%** | **A** | **B** | **C** | **D** | **E** | **F** | **G** | **H** | **I** |
|---|---|---|---|---|---|---|---|---|---|
| CPC-clay | 76.0 | 48.0 | 41.0 | 70.0 | 63.2 | 58.3 | 39.2 | 50.0 | 59.8 |
| Corn starch | - | - | - | - | - | - | - | - | 40.0 |
| Sorbitol | 24.0 | 22.0 | 19.0 | 20.0 | - | - | - | - | - |
| NaCl | - | 30.0 | 40.0 | - | - | - | - | - | - |
| SCMC | - | - | - | 4.0 | - | - | - | - | - |
| Abrasive silica | - | - | - | 6.0 | - | - | - | - | - |
| Eugragit RSPO | - | - | - | - | - | - | - | 50.0 | - |
| MCC | - | - | - | - | 18.0 | 20.0 | 20.0 | - | - |
| Ethyl cellulose | - | - | - | - | 18.0 | 20.0 | 40.0 | - | - |
| 20% w/v Kollidon^{®} VA 64 | - | - | - | - | 0.8 | 1.7 | 0.8 | - | 0.2 |

### Observations:

| | |
|---|---|
| A to D | : granules were not stable when included in a standard gel toothpaste |
| E | : moderately hard spherical noodles |
| F | : long string of noodles - granules curled up during spheronization |
| G | : powdery granules |
| H | : granules not formed |
| I | : rod shaped pellets formed |

When the granules/rods/noodles of Table 1 were used to make a series of gel toothpaste compositions (A1 to 11) respectively, it was observed that the granules/rods/noodles were unstable and lost their shape and structure. during short storage duration (within 10-15 days). Thus, none of the above composition were suitable for incorporation into a gel toothpaste composition. For details of a basic composition of gel toothpaste, reference may be made to Table 4.

### Example 4

An aim of this experiment also was to prepare granules, but all granular compositions of Table 2 were within scope of the present invention.

**TABLE 2**

| **Ingredients/wt%** | **1** | **2** | **3** | **4** | **5** | **6** | **7** | **8** |
|---|---|---|---|---|---|---|---|---|
| CPC-clay | 57.0 | 63.0 | 59.0 | 62.0 | 50.0 | 46.0 | 61.0 | 59.0 |
| Corn starch | 23.0 | - | 22.0 | 21.0 | - | | 19.0 | 18.0 |
| MCC | 5.0 | 5.0 | 4.0 | 5.0 | 8.0 | 8.0 | 9.5 | 9.0 |
| Eudragit NM 30 D | 15.0 | 11.0 | - | - | - | | - | 14.0 |
| Eudragit RSPO | - | 21.0 | - | - | 25.0 | | - | - |
| Ethyl cellulose dispersion Type B NF | - | - | 15.0 | 12.0 | - | | 10.5 | - |
| Eudragit NE 40D (40% w/w) | - | - | - | - | 17.0 | 23.0 | - | - |
| Ethyl Cellulose (Ethocel Standard 45) | - | - | - | - | - | 23.0 | - | - |

| | | | | | | | | |
|---|---|---|---|---|---|---|---|---|
| In the granular composition provided in table 2, CPC-clay is the bipolar antimicrobial particle, corn starch is the water-insoluble binder and the MCC, Eudragit NM 30 D, Eudragit RSPO, Ethyl cellulose dispersion Type B NF, Eudragit NE 40D (40% w/w), Ethyl cellulose (Ethocel Standard 45) were used as the water-insoluble coating agent. | | | | | | | | |

In each case, granules were formed at 1400 rpm for 20 minutes. The granules were spherical and their shape was retained even after five days in the granular composition of 1, 3, 4, 7 and 8. The composition 2, 5 and 6 having only coating agent and no binder was found to provide granular composition which were non-spherical. Physical properties of Composition 7 and 8 are listed below in Table 3. The granules of 7 and 8 were tested further as per Example 3.

**TABLE 3**

| **Property** | **Composition 7** | **Composition 8** |
|---|---|---|
| Bulk Density | 0.893 g/cm³ | 0.8771 q/ml |
| Friability | 0.70% w/w | 0.66% w/w |
| Particle size distribution | - | - |
| 1180 µ | 0.3% | 1180 µ |
| 1003 µ | 8.0% | 1003 µ |
| 850 µ | 61.7% | 850 µ |
| 710 µ | 22.45 % | 710 µ |
| 600 µ | 0.70 % | 600 µ |
| 250 µ & Fines | 6.67 % | 250 µ & Fines |
| Sphericity | 0.8 | 0.8 |

### Example 5: Gel toothpaste composition

All the granular compositions of Table 2 were used in the preparation of standard gel toothpaste formulations numbered as 1A to 8A (1A contained granular composition 1 of Table 2, and so on). Detailed formulation of the toothpaste is shown in Table 4.

**TABLE 4**

| **Ingredients** | **Wt%** |
|---|---|
| Sorbitol (70%) | 58.0 |
| Thickening Silica | 8.5 |
| Abrasive Silica (med) | 9.0 |
| Abrasive Silica (high) | 1.6 |
| SLS | 2.7 |
| granule** | 2.5 |
| SCMC 9H | 0.7 |
| NaOH solution (10%) | 0.3 |
| Water and others | 100.0 |

| | |
|---|---|
| ** refer Example 2 | |

In view of inclusion of the granular composition at 2.5 wt% in the toothpaste, the wt.% of the antimicrobial agent (quaternary ammonium compound, CPC) was estimated to be 0.04 % (in the toothpaste composition).

### Example 6

Two selected toothpaste compositions of Table 4 (7A and 8A) were picked up for further experiments. In this case, each toothpaste composition 7A and 8A was packaged in different collapsible tubes resulting in a total of 100 tubes (200 ml each) of toothpaste as per composition 7A and 100 other tubes containing 200 ml each of toothpaste as per 8A.

Of each set of 100 tubes, 5 tubes were selected randomly for the tests. Therefore, now there were two sets of tubes each containing 5 tubes. Samples of the toothpaste were drawn from each tube as follows:
- three samples from top section
- three samples from mid section
- three samples from bottom section

Each sample was tested for the CPC (cetyl pyridinium chloride) content (present in the granules). The expected amount of CPC was 0.04 wt% of the toothpaste composition. The following observations were recorded.

**TABLE 5**

| **Sample ref.** | **Actual wt% of CPC** | | |
|---|---|---|---|
| | **Top** | **Mid** | **Bottom** |
| Tube 1 (7A) | 0.040 | 0.029 | 0.045 |
| Tube 2 (7A) | 0.043 | 0.042 | 0.048 |
| Tube 3 (7A) | 0.045 | 0.031 | 0.043 |
| Tube 4 (7A) | 0.038 | 0.040 | 0.043 |
| Tube 5 (7A) | 0.040 | 0.033 | 0.045 |
| Tube 1 (8A) | 0.041 | 0.040 | 0.035 |
| Tube 2 (8A) | 0.041 | 0.053 | 0.031 |
| Tube 3 (8A) | 0.042 | 0.038 | 0.043 |
| Tube 4 (8A) | 0.039 | 0.038 | 0.033 |
| Tube 5 (8A) | 0.043 | 0.035 | 0.038 |

The data in Table 5 indicates that the observed value of CPC was almost constant across all three regions of the tubes, thereby indicating that there was no significant difference between middle, top & bottom layers of the composition (as in the pack) and the observed values did not deviate much from the calculated or expected value of 0.04 wt% (in the toothpaste). In other words, there was no spatial segregation into zones of high or poor concentration of the granules (and by that, the antimicrobial agent).

This demonstrates that when the granular composition according to the present invention having a bipolar antimicrobial particle and where the sphericity is in the range from 0.7 to 1.0, is incorporated in a personal care composition (toothpaste) the consumers receive the same amount of antimicrobial benefit in each dispense. Further these granular composition can also be perceived visually in the form of granules and also the granular composition further enhances the overall appearance of the toothpaste (personal care composition) while delivering functional benefits.

## Claims

1. A granular composition comprising 30 to 70 wt% bipolar antimicrobial particle, where said antimicrobial particle comprises a clay and a quaternary ammonium compound, in which said clay is an asymmetric 1:1 or a 2:1:1 clay having alternating tetrahedral and octahedral sheets, terminating with a tetrahedral sheet at one external surface plane and an octahedral sheet at another external surface plane, each said sheet comprising a co-ordinating cation; and said quaternary ammonium compound is attached to said coordinating cation on at least one external surface plane, wherein granules of said composition are spherical, having sphericity of 0.7 to 1.0.

2. A granular Z composition as claimed in claim 1 wherein average particle size of said granules is 650 to 900 µm.

3. A granular Z composition as claimed in claim 1 or 2 wherein bulk density of said granular composition is from 0.800 to 0.950 g/cm³.

4. A granular composition as claimed in any of claims 1 to 3 wherein said granules are friable and friability is from 0.6 to 0.9 w/w.

5. A granular composition as claimed in any of claims 1 to 4 wherein said composition comprises:
a) 10 to 40 wt% water-insoluble binder; and,
b) 5 to 23 wt% water-insoluble coating agent.

6. A granular composition as claimed in any of claims 1 to 5 wherein said quaternary ammonium compound is selected from cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide.

7. A granular composition as claimed in any of claims 1 to 6 wherein the antibacterial agent is attached to coordinating cations on the external surface of the octahedral surface plane.

8. A granular composition as claimed in any of claims 1 to 7 wherein said water-insoluble binder is a carbohydrate, and which carbohydrate is other than a cellulose or a cellulose derivative.

9. A granular composition as claimed in any of claims 1 to 8 wherein said water-insoluble coating agent is a polymer selected from the group consisting of [meth]acrylates, cellulose, or a derivative of cellulose or mixtures thereof.

10. A granular composition as claimed in any of claims 1 to 9 wherein, on w/w basis, said bipolar antimicrobial particle comprises 0.5 to 5 parts by weight of said quaternary ammonium compound and 90 to 98 parts by weight of said clay.

11. A granular composition as claimed in any of claims 1 to 10 wherein when said clay is an asymmetric 1:1 clay, it belongs to kaolinite or serpentine group of minerals.

12. A granular composition as claimed in any of claims 1 to 11 wherein when said clay is an an asymmetric 2:1 clay, it belongs to chlorite group of minerals.

13. A liquid personal care composition comprising granules of said granular composition as claimed in any of claims 1 to 12.

14. A liquid personal care composition as claimed in claim 13 wherein said personal care composition is a leave-on skin lotion, cream, antiperspirant, deodorant, serum, shampoo, hair conditioner, hair serum, hair gel, dentifrice, oral care composition, handwash, bodywash, shower gel or soap.

15. A liquid personal care composition as claimed in claim 14 wherein the dentifrice is a toothpaste composition.

## Patentansprüche

1. Granulatzusammensetzung, umfassend 30 bis 70 Gew.-% bipolare antimikrobielle Partikel, wobei die antimikrobiellen Partikel einen Ton und eine quaternäre Ammoniumverbindung umfassen, wobei der Ton ein asymmetrischer 1:1- oder ein 2:1:1-Ton mit abwechselnden tetraedrischen und oktaedrischen Schichten ist, die an einer Außenfläche mit einer tetraedrischen Schicht und an einer anderen Außenfläche mit einer oktaedrischen Schicht enden, wobei jede Schicht ein koordinierendes Kation umfasst und die quaternäre Ammoniumverbindung an mindestens einer Außenfläche an das koordinierende Kation gebunden ist, wobei die Granulate der Zusammensetzung kugelförmig sind und eine Sphärizität von 0,7 bis 1,0 aufweisen.

2. Granulatzusammensetzung nach Anspruch 1, wobei die durchschnittliche Partikelgröße der Granulate 650 bis 900 pm beträgt.

3. Granulatzusammensetzung nach Anspruch 1 oder 2, wobei die Schüttdichte der Granulat-Zusammensetzung zwischen 0,800 und 0,950 g/cm³ liegt.

4. Granulatzusammensetzung nach einem der Ansprüche 1 bis 3, wobei die Granulate bröckelig sind und die Bröckeligkeit zwischen 0,6 und 0,9 Gew.-% liegt.

5. Granulatzusammensetzung nach einem der Ansprüche 1 bis 4, wobei die Zusammensetzung
a) 10 bis 40 Gew.-% wasserunlösliches Bindemittel; und
b) 5 bis 23 Gew.-% wasserunlösliches Beschichtungsmittel umfasst.

6. Granulatzusammensetzung nach einem der Ansprüche 1 bis 5, wobei die quaternäre Ammoniumverbindung ausgewählt ist unter Cetylpyridiniumchlorid (CPC), Cetyltrimethylammoniumchlorid (CTAC), Cetyltrimethylammoniumbromid (CTAB), Benzalkoniumchlorid (BKC), Benzethoniumchlorid, Cetrimid, Quaternium, Tetrabutylammoniumbromid, Undecylenamidopropyltrimoniummethosulfat, Methylbenzethoniumchlorid, Cetethyldimoniumbromid, Cetromoniumtosylat, Cocotrimoniumchlorid, Dodecylbenzyltrimoniumchlorid, Laurylisochinoliumbromid, Laurylpyridiniumchlorid, Dequaliniumchlorid oder Domiphenbromid.

7. Granulatzusammensetzung nach einem der Ansprüche 1 bis 6, wobei das antibakterielle Mittel an koordinierenden Kationen an der Außenfläche der oktaedrischen Oberflächenebene gebunden ist.

8. Granulatzusammensetzung nach einem der Ansprüche 1 bis 7, wobei das wasserunlösliche Bindemittel ein Kohlenhydrat ist, wobei das Kohlenhydrat kein Cellulose oder Cellulosederivat ist.

9. Granulatzusammensetzung nach einem der Ansprüche 1 bis 8, wobei das wasserunlösliche Beschichtungsmittel ein Polymer ist, das aus der Gruppe ausgewählt ist, die aus [Meth]acrylaten, Cellulose oder einem Derivat von Cellulose oder Mischungen davon besteht.

10. Granulatzusammensetzung nach einem der Ansprüche 1 bis 9, wobei auf Gewichtsbasis das bipolare antimikrobielle Partikel 0,5 bis 5 Gewichtsteile der quaternären Ammoniumverbindung und 90 bis 98 Gewichtsteile des Tons umfasst.

11. Granulatzusammensetzung nach einem der Ansprüche 1 bis 10, wobei, wenn der Ton ein asymmetrischer 1:1-Ton ist, dieser zur Kaolinit- oder Serpentin-Gruppe der Mineralien gehört.

12. Granulatzusammensetzung nach einem der Ansprüche 1 bis 11, wobei, wenn der Ton ein asymmetrischer 2:1-Ton ist, dieser zur Chloritgruppe der Mineralien gehört.

13. Flüssige Körperpflegezusammensetzung, die Granulate der Granulat-Zusammensetzung nach einem der Ansprüche 1 bis 12 umfasst.

14. Flüssige Körperpflegezusammensetzung nach Anspruch 12, wobei die Körperpflegezusammensetzung eine Leave-on-Hautlotion, Creme, ein Antitranspirant, Deodorant, Serum, Shampoo, Haarkonditionierer, Haarserum, Haargel, Zahnpasta, Mundpflegezusammensetzung, Handwaschmittel, Körperwaschmittel, Duschgel oder Seife ist.

15. Flüssige Körperpflegezusammensetzung nach Anspruch 14, wobei die Zahnpasta eine Zahncremezusammensetzung ist.

## Revendications

1. Composition granulaire comprenant 30 à 70 % en poids de particules antimicrobiennes bipolaires, où lesdites particules antimicrobiennes comprennent une argile et un composé d'ammonium quaternaire, dans laquelle ladite argile est une argile 1/1 ou 2/1/1 asymétrique ayant des feuillets tétraédriques et octaédriques alternés, se terminant par un feuillet tétraédrique au niveau d'un plan de surface externe et un feuillet octaédrique au niveau d'un autre plan de surface externe, chaque dit feuillet comprenant un cation de coordination ; et ledit composé d'ammonium quaternaire est rattaché audit cation de coordination sur au moins un plan de surface externe, dans laquelle les granules de ladite composition sont sphériques, en ayant une sphéricité de 0,7 à 1,0.

2. Composition granulaire selon la revendication 1, dans laquelle la granulométrie moyenne desdits granules est de 650 à 900 µm.

3. Composition granulaire selon la revendication 1 ou 2, dans laquelle la masse volumique apparente de ladite composition granulaire est de 0,800 à 0,950 g/cm³.

4. Composition granulaire selon l'une quelconque des revendications 1 à 3, dans laquelle lesdits granules sont friables et la friabilité est de 0,6 à 0,9 w/w.

5. Composition granulaire selon l'une quelconque des revendications 1 à 4, laquelle composition comprend
a) 10 à 40 % en poids de liant insoluble dans l'eau ; et
b) 5 à 23 % en poids d'agent de revêtement insoluble dans l'eau.

6. Composition granulaire selon l'une quelconque des revendications 1 à 5, dans laquelle ledit composé d'ammonium quaternaire est choisi parmi le chlorure de cétylpyridinium (CPC), le chlorure de cétyltriméthylammonium (CTAC), le bromure de cétyltriméthylammonium (CTAB), le chlorure de benzalkonium (BKC), le chlorure de benzéthonium, le cétrimide, le quaternium, le bromure de tétrabutylammonium, le méthosulfate d'undécylène-amidopropyltrimonium, le chlorure de méthylbenzéthonium, le bromure de cététhyldimonium, le tosylate de cétromonium, le chlorure de (coprah-yl)trimonium, le chlorure de dodécylbenzyltrimonium, le bromure de laurylisoquinolium, le chlorure de laurylpyridinium, le chlorure de déqualinium et le bromure de domiphène.

7. Composition granulaire selon l'une quelconque des revendications 1 à 6, dans laquelle l'agent antibactérien est rattaché à des cations de coordination sur la surface externe du plan de surface octaédrique.

8. Composition granulaire selon l'une quelconque des revendications 1 à 7, dans laquelle ledit liant insoluble dans l'eau est un hydrate de carbone, lequel hydrate de carbone est autre qu'une cellulose ou qu'un dérivé de cellulose.

9. Composition granulaire selon l'une quelconque des revendications 1 à 8, dans laquelle ledit agent de revêtement insoluble dans l'eau est un polymère choisi dans le groupe constitué par les [méth]acrylates, la cellulose, un dérivé de cellulose, et leurs mélanges.

10. Composition granulaire selon l'une quelconque des revendications 1 à 9, dans laquelle, sur une base en poids/poids, lesdites particules antimicrobiennes bipolaires comprennent 0,5 à 5 parties en poids dudit composé d'ammonium quaternaire et 90 à 98 parties en poids de ladite argile.

11. Composition granulaire selon l'une quelconque des revendications 1 à 10, dans laquelle, quand ladite argile est une argile 1/1 asymétrique, elle appartient au groupe de minéraux de la kaolinite ou de la serpentine.

12. Composition granulaire selon l'une quelconque des revendications 1 à 11, dans laquelle, quand ladite argile est une argile 2/1 asymétrique, elle appartient aux groupes de minéraux de la chlorite.

13. Composition de soin personnel liquide comprenant des granules de ladite composition granulaire de l'une quelconque des revendications 1 à 12.

14. Composition de soin personnel liquide selon la revendication 13, laquelle composition de soin personnel liquide est une lotion pour la peau sans rinçage, une crème, un antitranspirant, un déodorant, un sérum, un shampooing, un conditionneur capillaire, un sérum capillaire, un gel capillaire, un dentifrice, une composition d'hygiène buccale, un produit de lavage des mains, un produit de lavage du corps, un gel douche ou un savon.

15. Composition de soin personnel liquide selon la revendication 14, dans laquelle le dentifrice est une composition de pâte dentifrice.
